(19) Europäisches Patentamt

European Patent Office

Office européen des brevets

(11) **EP 2 077 727 B1**

(12) **EUROPEAN PATENT SPECIFICATION**

(45) Date of publication and mention
of the grant of the patent:
**15.06.2011 Bulletin 2011/24**

(21) Application number: **06791432.5**

(22) Date of filing: **29.09.2006**

(51) Int Cl.:
***A23G 4/00*** *(2006.01)* ***A61K 9/00*** *(2006.01)*

(86) International application number:
**PCT/DK2006/000540**

(87) International publication number:
**WO 2008/037252 (03.04.2008 Gazette 2008/14)**

(54) **CHEWING GUM COMPRISING HVDROXYAPATITE**

HYDROXYAPATITHALTIGER KAUGUMMI

CHEWING-GUM COMPRENANT DE L'HYDROXYAPATITE

(84) Designated Contracting States:
**AT BE BG CH CY CZ DE DK EE ES FI FR GB GR
HU IE IS IT LI LT LU LV MC NL PL PT RO SE SI
SK TR**

(43) Date of publication of application:
**15.07.2009 Bulletin 2009/29**

(73) Proprietor: **Gumlink A/S**
**7100 Vejle (DK)**

(72) Inventors:
• **PORSGAARD, Tania Kjølhede**
**8220 Brabrand (DK)**

• **GYLDENVANG, Lars**
**8500 Grenå (DK)**

(74) Representative: **Kitchen, Steven Richard**
**Chas. Hude A/S**
**H.C. Andersens Boulevard 33**
**1780 Copenhagen V (DK)**

(56) References cited:
**WO-A-98/18719 DE-A1- 2 134 862
US-A1- 2004 171 471**

**Description**

Technical Field

The present invention relates to solid oral compositions.

In a particular embodiment the invention relates to a chewing gum.

The invention further relates to the use of such compositions to prevent sensitive teeth or to remineralise or whiten teeth.

Background Art

Hydroxyapatite ($Ca_{10}(PO_4)_6(OH)_2$) is chemically similar to the mineral component of bones and tooth enamel in mammals. It is one of few materials that are classed as bioactive, meaning that it will support bone ingrowth and tooth remineralisation when used in orthopaedic, dental and maxillofacial applications.

Tooth paste compositions comprising hydroxyapatite are known to have beneficial properties when applied on teeth, by filling pores and spaces of the teeth surface forming a light solid film on the surface of the teeth. Among other things this causes relief from hypersensitivity. Hypersensitivity is caused by various mechanical, e.g. thermal, evaporative and osmotic stimuli, which cause rapid outward flow of fluid in the dentinal tubules, the fluid flow creates a pressure change across the dentin, stimulating the nerve fibre and resulting in the perception of pain. Especially individuals wherein the gingival line has moved up exposing parts of the root of the teeth are known to experience hypersensitivity. This is due to the fact that whereas the crown of the teeth (the crown is the section of tooth normally exposed to the interior of the mouth; the section below the gingival line is the root) is covered with enamel, the hardest substance in the human body, the root is covered with a softer substance known as cementum. Beneath the enamel and cementum is a material known as dentin, which encloses the tooth's sensory mechanisms, such as the dental pulp or nerve root. Dentin is 70% inorganic material, 18% organic material, and 12% water. The dentin is riddled with thousands of small channels known as dentinal tubules. All of the 12% fluid in the dentine is located almost completely within the tubules. The diameter of the tubules averages 2.5 microns at the pulp and 0.8 microns at cementum side. The tubules are devoid of nerve tissue except for very short extensions. The nerve cell bodies reside in the pulp and sense only pain.

Hydroxyapatites in nano-scale crystals are highly hydrophilic and they have a large surface area, hence these crystals are hygroscopic and their surface is always moist. When applied to a surface e.g. tooth surface, they produce a thin, but strong, liquid film, which binds to the tooth crown. In the thin film the crystals are self-organised in a grid of positively and negatively charged sites, which results in a relative disorganised surface structure of the nano-crystal. The great energy gain is produced by a parallel arrangement of the surface such that positive and negative sites always interact with each other. Because the nano-crystals are rod shaped the grid of crystals can be even tighter densely packed. This tightly packed film lies on top of the tubules in the dentin enamel reducing the sensitivity, because the stimuli are being blocked by the thin film. This thin film also whitens the teeth, because the appearance of the film is white. It has also been shown to have a remineralising effect, because of the high content of calcium and phosphate in the ingredient, and these ingredients have previously been shown to remineralised the tooth enamel of humans. (Pickel FD, Bilotti A. The effects of a chewing gum containing dicalcium phosphate on salivary calcium and phosphate Ala. J. Med. Sci. 1965; 2: 3. 288-293)

The use of hydroxyapatite in liquid or flowable oral compositions, e.g. toothpaste, for the purpose of protecting against sensitive teeth is known. However, formulation of solid oral compositions containing hydroxyapatite, wherein the hydroxyapatite is released in the oral cavity after ingestion of the composition, has proven difficult, as the hydroxyapatite, when entering the oral cavity, are retained in the solid components of the compositions, and consequently the crystals are not released to perform its desired action on the surface of the teeth.

Document WO98/18719 A discloses a chewing gum comprising hydroxylapatite with length-to-breath ratio of 5 to 50.

Disclosure of Invention

Surprisingly, it has now been shown that solid oral compositions containing hydroxyapatite, which compositions are useful for treating and preventing sensitive teeth as well as remineralising and whiten teeth can be manufactured.

Accordingly, the present invention relate to a solid oral composition comprising:

a) at least one gum base system,
b) at least one chewing gum additive,
c) rod-shaped apatite crystals of the formula $Ca_5(PO_4)_3(OH)_xF_y$ having the following features i) the length-to-breadth ratio of the crystals is at least $\geq 5$ and ii) $x+y=1$, where if x or $y \neq 0$ the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions,

characterised in that the concentration of said crystals is higher in the chewing gum additive part of the solid oral composition than in gum base system part of the solid oral composition.

In a preferred embodiment, the solid oral composition is a chewing gum.

In one embodiment, the apatite crystals are present in the composition in an amount of between 0.01 % and 30% by weight of the solid oral composition.

The invention further relates to a method of providing a solid oral composition comprising rod-shaped apatite crystals of the formula $Ca_5(PO_4)_3(OH)_xF_y$ having the following features i) the length-to-breadth ratio of the crystals is at least ≥5 and ii) x+y=1, where if x or y≠0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions, said solid oral composition being capable of releasing said crystals in the oral cavity, which method comprises the steps of i) mixing said crystals with at least one chewing gum additive into a homogenous premix ii) mixing said crystals with remaining chewing gum additives and gum base system.

By pre-mixing the hydroxyapatite crystals with the water-soluble additives of the solid composition, a better release of the hydroxyapatite is obtained. Further, a better release may be obtained by pre-mixing the hydroxyapatite as powdered crystals.

In a further embodiment the invention relates to the use of a composition according to the invention for remineralising teeth.

Furthermore, the present invention relates to a method of remineralising teeth.

In a further embodiment the invention relates to the use of a composition according to the invention for preventing sensitive teeth.

Furthermore, the present invention relates to a method of preventing sensitive teeth.

In a further embodiment the invention relates to the use of a composition according to the invention for whitening teeth.

Furthermore, the present invention relates to a method of whitening tooth surfaces.

**Definitions**

By "solid oral composition" is meant a solid composition for oral use such as a confectionary composition e.g. hard candy, hard-boiled candy, soft candy such as gummy confectionary and jellies, cotton candy, compressed or pressed tablets, chewing gum, film, lozenges, chocolate, center-filled confectioneries, such as gel or liquid filled confectioneries in any shape. Solid oral compositions may comprise a coating. When referring to the weight of a solid oral composition according to the invention the intention is to refer to the weight excluding coating unless otherwise indicated.

By the phrase "chewing gum" is meant any chewing gum such as conventional chewing gum, centre-filled chewing gum, liquid filled chewing gum, toffee imitating chewing gum or compressed chewing gum (or compressed chewing gum tablet).

"Hydroxyapatite" ($Ca_5(OH)(PO_4)_3$) is often referred to as "hydroxylapatite" or HAP. Hydroxyapatite having a size as preferred according to the present invention may also be referred to as nano-sized crystal hydroxyapatite or nano-HAP. The hydroxyapatite crystals according to the invention may also comprise hydroxyapatite derivatives. A hydroxyapatite derivative may be any derivative of hydroxyapatite, e.g. fluorinated hydroxyapatite.

Best Modes for Carrying out the Invention

It has now surprisingly been demonstrated that solid oral compositions containing hydroxyapatite crystals can be formulated by adding nano-sized hydroxyapatite crystals to the water soluble part of the solid oral compositions, prior to any contact to the water insoluble parts of the compositions.

Preferred hydroxyapatite crystals according to the invention are described in US2004171471A.

Preferably, the hydroxyapatite crystals according to the invention are rod-shaped apatite crystals of the formula $Ca_5(PO_4)_3(OH)_xF_y$ having the following features i) the length-to-breadth ratio of the crystals is at least ≥5 and ii) x+y=1, where if x or y≠0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions.

Most preferably, the hydroxyapatite crystals according to the invention are rod-shaped apatite crystals of the formula ($Ca_5(OH)(PO_4)_3$) wherein the length-to-breadth ratio of the crystals is at least ≥5.

Preferably, hydroxyapatite crystals according to the invention are present in the composition according to the invention in an amount of between 0.05% and 20% by weight of the solid oral composition.

More preferably, hydroxyapatite crystals are present in the compositions in an amount of between 0.1 % and 15 by weight of the solid oral composition.

Even more preferably, hydroxyapatite crystals are present in the compositions in an amount of between 0.5% and 10% by weight of the solid oral composition.

Even more preferably, hydroxyapatite crystals are present in the compositions in an amount of between 0.5% and 5% by weight of the solid oral composition.

Preferably, the hydroxyapatite crystals according to the invention have a mean particle size of between 0.01 and 0.2 μm measured as length.

In a particular embodiment according to the invention, the hydroxyapatite crystals are rod shaped and have a length-to-breadth ratio of more than 7.

Preferably, the hydroxyapatite or derivates thereof are added to the compositions as powdered crystals. In a preferred embodiment the hydroxyapatite or derivates thereof are added as powdered nano-sized crystals.

The compositions of the invention are essentially solid and comprise more than 75%, preferably more than 85%, even more preferably more than 95%, by weight of the composition of solid materials.

In one embodiment, the composition according to the invention may be formulated as a chewing gum composition, said chewing gum composition preferably comprising a gum base system constituting from 10% to 99%, particularly from 15% to 80%, preferably 20% to 60 % by weight of the composition.

*Gum base*

As used herein, the expression "gum base system" refers in general to the water insoluble part of the chewing gum which typically constitutes 10 to 99% by weight including the range of 15-50% by weight of the total chewing gum formulation. Chewing gum base systems typically comprise one or more elastomeric compounds which may be of synthetic or natural origin, one or more resin compounds which may be of synthetic or natural origin, fillers, softening compounds and minor amounts of miscellaneous ingredients such as antioxidants and colorants, etc.

According to a preferred embodiment of the invention, the gum base should comprise at least an amount of 5% by weight of natural resins.

The composition of chewing gum base systems which are admixed with chewing gum additives as defined below can vary substantially depending on the particular product to be prepared and on the desired masticatory and other sensory characteristics of the final product.

However, typical ranges by weight of the above gum base system components are: 5 to 50% by weight of elastomer, 5 to 55% by weight of plasticizer, 0 to 50% by weight of filler/texturiser, 5 to 35% by weight of softener and 0 to 1 % by weight of miscellaneous ingredients such as antioxidants, colourants, etc.

According to a preferred embodiment of the invention, the gum base is partly premixed with a moderate amount of flavour and/or active ingredients.

In this context, useful synthetic elastomers include, but are not limited to, synthetic elastomers listed in Food and Drug Administration, CFR, Title 21, Section 172,615, the Masticatory Substances, Synthetic) such as polyisobutylene with a gel permeation chromatography (GPC) average molecular weight in the range of about 10,000 to about 1,000,000 including the range of 50,000 to 80,000, isobutylene-isoprene copolymer (butyl elastomer), styrene-butadiene copolymers e.g. having styrene-butadiene ratios of about 1:3 to about 3:1, polyisoprene, polyethylene, vinyl acetate-vinyl laurate copolymer e.g. having a vinyl laurate content of about 5 to about 50% by weight such as 10 to 45% by weight of the copolymer, and combinations hereof.

Useful natural non-degradable elastomers include the elastomers listed in Food and Drug Administration, CFR, Title 21, Section 172,615, as "Masticatory Substances of Natural Vegetable Origin" including natural rubber compounds such as smoked or liquid latex and guayule and other natural gums including jelutong, lechi caspi, massaranduba balata, sorva, perillo, rosindinha, massaranduba chocolate, chicle, nispero, gutta hang kang, and combinations thereof. The preferred synthetic elastomer and natural elastomer concentrations vary depending on whether the chewing gum in which the base is used is adhesive or conventional, bubble gum or regular gum. Presently preferred natural elastomers include jelutong, chicle, massaranduba balata and sorva.

When the chewing gum according to the invention comprises about 0.5% to 30% by weight of elastomers, preferably about 5% to 25% by weight, a further advantageous embodiment of the invention has been obtained.

Plasticizers in conventional chewing gum base systems typically include synthetic resins such as poly(vinyl acetate) (PVAc) and natural resins such as rosin esters which are often referred to as ester gums. Additionally, natural resins such as glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins are typically applied in chewing gum bases. Other resinous compounds typically applied in chewing gum bases include synthetic resins such as terpene resins derived from alpha-pinene, beta-pinene, and/or d-limonene and natural terpene resins.

According to the invention, it has been recognized that a natural resin facilitates an advantageous overall flavour release when the oral composition is chewed. This may partly be due to the fact that the initial chewing of the composition results in an immediate release of distinct flavour particles and at the same time, that a part of the dissolved flavour particles react or become incorporated into the chewing gum base.

When the chewing gum according to the invention comprises about 3 % to 50 % by weight of resins, preferably about 4% to 30% by weight, an advantageous embodiment of the invention may been obtained.

When the natural resin comprises rosin esters, a further advantageous embodiment of the invention may been obtained.

When said natural resin comprises glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins or pentaerythritol esters of rosins, a further advantageous embodiment of the invention has been obtained.

Preferably, the gum base system comprises about 8% to 40% by weight of resin.

In one embodiment, the use of natural resins is preferred in the gum base system is preferred.

Evidently, according to a further embodiment of the invention, the natural resin may be supplemented by a synthetic resin, such as PVA.

A chewing gum base system may, if desired, include one or more fillers/texturisers including as examples, magnesium and calcium carbonate, sodium sulphate, ground limestone, silicate compounds such as magnesium and aluminium silicate, kaolin and clay, aluminium oxide, silicium oxide, talc, titanium oxide, mono-, di- and tri-calcium phosphates, cellulose polymers, such as wood, and combinations thereof.

A gum base system may, in accordance with the present invention comprise one or more softening agents e.g. sucrose polyesters including those disclosed in WO 00/25598, tallow, hydrogenated tallow, hydrogenated and partially hydrogenated vegetable oils, cocoa butter, glycerol monostearate, glycerol triacetate, lecithin, mono-, di- and triglycerides, acetylated monoglycerides, fatty acids (e.g. stearic, palmitic, oleic and linoleic acids), and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like are examples of conventionally used emulsifiers which can be added to the chewing gum base. In case of the presence of a biologically or pharmaceutically active ingredient as defined below, the formulation may comprise certain specific emulsifiers and/or solubilisers in order to disperse and release the active ingredient.

Waxes and fats are conventionally used for the adjustment of the consistency and for softening of the chewing gum base when preparing chewing gum bases. In connection with the present invention any conventionally used and suitable type of wax and fat may be used, such as for instance rice bran wax, polyethylene wax, petroleum wax (refined paraffin and microcrystalline wax), paraffin, bees' wax, carnauba wax, candelilla wax, cocoa butter, degreased cocoa powder and any suitable oil or fat, as e.g. completely or partially hydrogenated vegetable oils or completely or partially hydrogenated animal fats.

In one embodiment the gum base is wax-free.

Furthermore, the gum base formulation may, in accordance with the present invention, comprise colourants and whiteners such as FD&C-type dyes and lakes, fruit and vegetable extracts, titanium dioxide and combinations thereof. Further useful chewing gum base components include antioxidants, e.g. butylated hydroxytoluene (BHT), butyl hydroxyanisol (BHA), propylgallate and tocopherols, and preservatives.

*Chewing gum additives*

As used herein the expression "chewing gum additives" or "gum additives" refers in general to the water soluble part of the solid oral composition. Chewing gum additives include bulk sweeteners, high intensity sweeteners, taste enhancers, flavouring agents, softeners, emulsifiers, colouring agents, binding agents, acidulants, fillers, antioxidants and other components such as pharmaceutically or biologically active substances, that confer desired properties to the finished chewing gum product.

Sweeteners, high intensity sweeteners and taste enhancers are well known to the skilled person. Non-limiting examples of sweeteners comprise sugar sweeteners including saccharides such as sucrose, dextrose, glucose, maltose, dextrins, D-tagatose, trehalose, dried invert sugar, fructose, levulose, galactose, corn syrup solids, and the like, alone or in combination. Other examples of sweeteners comprise sugarless sweeteners including polyhydric alcohols such as sorbitol, mannitol, xylitol, glycerol, hydrogenated starch hydrolysates, maltitol, isomaltitol, erythritol, lactitol and the like, alone or in combination. Sugarless sweeteners are preferred.

Preferred high intensity sweeteners include but are not limited to sucralose, aspartame, salts of acesulfame, alitame, saccharin or salts thereof, neotame, cyclamic acid and salts thereof, glycyrrhizin, dihydrochalcones thaumatin, monnelin, sterioside and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

Examples of suitable sweeteners are listed below.

Suitable bulk sweeteners include e. g. both sugar and non-sugar components. Bulk sweeteners typically constitute from about 5 to about 95% by weight of the solid oral composition, more typically about 20 to about 80% by weight such as 30 to 60% by weight of the gum.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

High intensity artificial sweetening agents can also be used alone or in combination with the above sweeteners. Preferred high intensity sweeteners include, but are not limited to sucralose, aspartame, salts of acesulfame, alitame, saccharin and its salts, neotam, cyclamic acid and its salts, glycyrrhizin, dihydrochalcones, thaumatin, monellin, sterioside and the like, alone or in combination. In order to provide longer lasting sweetness and flavour perception, it may be desirable to encapsulate or otherwise control the release of at least a portion of the artificial sweetener.

Usage level of the artificial sweetener will vary considerably depending e. g. on factors such as potency of the sweetener, rate of release, desired sweetness of the product, level and type of flavour used and cost considerations. Thus, the active level of artificial sweetener may vary from about 0.02 to about 8% by weight. When carriers used for encapsulation are included, the usage level of the encapsulated sweetener will be proportionately higher. Combinations of sugar and/or non-sugar sweeteners can be used in the solid oral composition formulation processed in accordance with the invention.

In one further embodiment, sucrose fatty acid esters may also be utilised for increased release of sweeteners including for instance the so-called highly potent sweeteners, such as for instance saccharin, cyclamate, aspartame, thaumatin, dihydrocalcones, stevioside, glycyrrhizin or salts or compounds thereof. For increased released of sweetener, the sucrose fatty acids preferable have a content of palmitate of at least 40% such as at least 50%.

Additionally, the softener may also provide additional sweetness such as with aqueous sugar or alditol solutions.

If a low calorie gum is desired, a low caloric bulking agent can be used. Examples of low caloric bulking agents include polydextrose, Raftilose, Raftilin, Inuline, fructooligosaccharides (NutraFlora), palatinose oligosaccharided; guar gum hydrolysates (e. g. Sun Fiber@) or indigestible dextrins (e. g. Fibersol@). However, other low calorie-bulking agents can be used.

Preferably, the compositions according to the invention comprise a flavour. A variety of flavours known in the art may be used, such as cinnamon, wintergreen, eucalyptus, spearmint, peppermint, menthol, anise as well as fruit flavours such as apple, pear, peach, strawberry, cherry, apricot, orange, watermelon, banana and the like; bean-derived flavours, such as coffee, cocoa and the like. Flavouring agents are incorporated in the solid oral composition formulation at a concentration of about 0.5 to about 5 % by weight and preferably 1 to 3 % by weight.

Aroma agents and flavouring agents which are useful in a solid oral composition produced by the present process are e. g. natural and synthetic flavourings (including natural flavourings) in the form of freeze-dried natural vegetable components, essential oils, essences, extracts, powders, including acids and other substances capable of affecting the taste profile. Examples of liquid and powdered flavourings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In one preferred embodiment, the flavour is one or more natural flavouring agent (s) which is/are freeze-dried, preferably in the form of a powder, slices or pieces of combinations thereof. The particle size of such agent may be less than 3 mm, such as less than 2 mm, more preferred less than 1 mm, calculated as the longest dimension of the particle. The natural flavouring agent may also be in a form where the particle size is from about 3 um to 2 mm, such as from 4 um to 1 mm. Preferred natural flavouring agents include seeds from a fruit e. g. from strawberry, blackberry and raspberry.

Various synthetic flavours, such as mixed fruit flavour may also be used according to the present invention. As indicated above, the aroma agent may be used in quantities smaller than those conventionally used. The aroma agents and/or flavours may be used in an amount of from 0.01 to about 30% by weight of the final product depending on the desired intensity of the aroma and/or flavour used. Preferably, the content of aroma/flavour is in the range of from 0.2 to 3% by weight of the total composition.

Different methods of encapsulating flavours or active ingredients, which may both refer to flavours or active ingredients mixed into the gum base and flavours or active ingredients mixed into the solid oral composition may e. g. include Spray drying, Spray cooling, Film coating, Coascervation, Double emulsion method (Extrusion technology) or Prilling Materials to be used for the above mentioned encapsulation methods may e. g. include Gelatine, Wheat protein, Soya protein, Sodium caseinate, Caseine, Gum arabic, Mod. starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

When the solid oral composition comprises about 0.1 % to 15 % by weight of flavouring agents, preferably about 0. 8 % to 5 % by weight, a further advantageous embodiment of the invention has been obtained.

The amount of flavour depends heavily of the applied type of flavour and the method of application as well as the gum type of solid oral composition.

Further chewing gum additives which may be included in the solid oral composition include surfactants and/or solubilisers, especially when pharmaceutically, cosmetically or biologically active ingredients are present. As examples of types of surfactants to be used as solubilisers in a solid oral composition composition according to the invention reference is made to H. P. Fiedler, Lexikon der Hilfstoffe fr Pharmacie, Kosmetik und Angrenzende Gebiete, page 63-64 (1981) and the lists of approved food emulsifiers of the individual countries.

In one embodiment, the solid oral composition comprises a solubiliser. Anionic, cationic, amphoteric or non-ionic solubilisers can be used. Suitable solubilisers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hy- drogenated castor oil (e. g. the product sold under the trade name CREMOPHOR), block copolymers of ethylene oxide and propylene oxide (e. g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene (8) stearate and polyoxyethylene (40) stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds. The expression "solubiliser" is used in the present text to describe both possibilities, the solubiliser used must be suitable for use in food and/or medicine.

It may be advantageous to include one or more additional tooth whitening agents. Examples of such additional tooth whitening agents are well known in the art and include abrasives as well as bleaching agents. Abrasive materials comprise as non-limiting examples silica, alumina, calcium carbonate, dicalcium phosphate, calcium pyrophosphate, trimetaphosphates and insoluble hexametaphosphates. Bleaching agents comprise agents such as peroxy compounds, e.g. potassium peroxydiphosphate and urea-peroxid. Effervescing systems such as sodium bicarbonate, alone or in combination with citric acid as well as colour change systems may also be incorporated into compositions comprised by the present invention.

In one embodiment, the solid oral composition according to the invention comprises a pharmaceutically, cosmetically or biologically active substance. Examples of such active substances, a comprehensive list of which is found e. g. in WO 00/25598, include drugs, dietary supplements, antiseptic agents, pH adjusting agents, anti-smoking agents and substances for the care or treatment of the oral cavity and the teeth such as oral hygiene promoting agents, anti-calculus agents, anti-caries agents, anti-microbial agents, anti-inflammatory agents, desensitising agents, remineralising agents, hydrogen peroxide and compounds capable of releasing urea during chewing.

In one embodiment the solid oral composition according to the invention comprises one or more therapeutically active agents. Non-limiting examples comprise anti-caries agents such as sodium, calcium, magnesium and stannous fluoride, amine fluorides, disodium monofluorophosphate, sodium trimetaphosphate and casein; antimicrobial agents, e.g. Triclosan, chlorhexidine, copper, zinc and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol); anti-inflammatory agents such as ibuprofen, flurbiprofen, aspirin, indomethacin etc.; plaque acid buffers such as urea, calcium lactate, calcium glycerophosphate and strontium polyacrylates; desensitising agents, e.g. potassium citrate, potassium chloride, potassium tartrate, potassium bicarbonate, potassium oxalate, potassium nitrate and strontium salts; anti-calculus agents, e.g.

hypophosphite-containing polymers, organic phosphonates and phosphocitrates etc.; gum protection agents, e.g. vegetable oils such as sunflower oil, rape seed oil, soybean oil, safflower oil; silicone oil; and hydrocarbon oil; pharmaceutically acceptable carriers, e.g. starch, sucrose, water or water/alcohol systems etc.; surfactants, such as anionic, nonionic, cationic and zwitterionic or amphoteric surfactants.

Other agents which may be incorporated in the solid oral compositions of the present invention are agents to counter breath malodour and include water soluble zinc salts (at least 1 % soluble) particularly zinc chloride, zinc acetate, zinc citrate and zinc gluconate.

Examples of further suitable active ingredients are listed below.

Examples of useful active substances in the form of antiseptics include salts and derivatives of guanidine and biguanidine (for instance chlorhexidine diacetate) and the following types of substances with limited water-solubility: quaternary ammonium compounds (e. g. ceramine, chloroxylenol, crystal violet, chloramine), aldehydes (e. g. paraformaldehyde), derivatives of dequaline, polynoxyline, phenols (e. g. thymol, p-chlorophenol, cresol), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, The Extra Pharmacopoeia, 28th edition, page 547-578; metal salts, complexes and compounds with limited water-solubility, such as aluminium salts, (for instance aluminium potassium sulphate AlK $(SO_4)_2$, $12H_2O$) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodium monofluorophosphate, aminofluorides, stannous fluoride), phosphates, carbonates and selenium. Further active substances can be found in J. Dent. Res. Vol. 28 No. 2, page 160-171,1949.

Examples of active substances in the form of agents adjusting the pH in the oral cavity include: acids, such as adipic acid, succinic acid, fumaric acid, or salts thereof or salts of citric acid, tartaric acid, malic acid, acetic acid, lactic acid, phosphoric acid and glutaric acid and acceptable bases, such as carbonates, hydrogen carbonates, phosphates, sulphates or oxides of sodium, potassium, ammonium, magnesium or calcium, especially magnesium and calcium.

Active ingredients may comprise the below mentioned compounds or derivates thereof but are not limited thereto: Acetaminophen, Acetylsalicylsyre Buprenorphine Bromhexin Celcoxib Codeine, Diphenhydramin, Diclofenac, Etoricoxib, Ibuprofen, Indometacin, Ketoprofen, Lumiracoxib, Morphine, Naproxen, Oxycodon, Parecoxib, Piroxicam, Pseudoefedrin, Rofecoxib, Tenoxicam, Tramadol, Valdecoxib, Calciumcarbonat, Magaldrate, Disulfiram, Bupropion, Nicotine, Azithromycin, Clarithromycin, Clotrimazole, Erythromycin, Tetracycline, Granisetron, Ondansetron, Prometazin, Tropisetron, Brompheniramine, Ceterizin, leco-Ceterizin, Chlorcyclizine, Chlorpheniramin, Chlorpheniramin, Difenhydramine, Doxylamine, Fenofenadin, Guaifenesin, Loratidin, des-Loratidin, Phenyltoloxamine, Promethazin, Pyridamine, Terfenadin, Troxerutin, Methyldopa, Methylphenidate, Benzalcon Chloride, Benzeth. Chloride, Cetylpyrid. Chlorhexidine, Ecabet-sodium, Haloperidol, Allopurinol, Colchinine, Theophylline, Propanolol, Prednisolone, Prednisone, Fluoride, Urea, Actot, Glibenclamide, Glipizide, Metformin, Miglitol, Repaglinide, Rosiglitazone, Apomorfin, Cialis, Sildenafil, Vardenafil, Diphenoxylate, Simethicone, Cimetidine, Famotidine, Ranitidine, Ratinidine, cetrizin, Loratadine, Aspirin, Benzocaine, Dextrometorphan, Phenylpropanolamine, Pseudoephedrine, Cisapride, Domperidone, Metoclopramide, Acyclovir, Dioctylsulfosucc., Phenolphtalein, Almotriptan, Eletriptan, Ergotamine, Migea, Naratriptan, Rizatriptan, Sumatriptan, Zohnitriptan, Aluminium salts, Calcium salts, Ferro salts, Silver salts, Zinc-salts, Amphotericin B, Chlorhexidine, Miconazole, Triamcinolonacetonid, Melatonine, Phenobarbitol, Caffeine, Benzodiazepiner, Hydroxyzine, Meprobamate, Phenothiazine, Buclizine, Brometazine, Cinnarizine, Cyclizine, Difenhydramine, Dimenhydrinate, Buflomedil, Amphetamine, Caffeine, Ephedrine, Orlistat, Phenylephedrine, Phenylpropanolamin, Pseudoephedrine, Sibutramin, Ketoconazole, Nitroglycerin, Nystatin, Progesterone, Testosterone, Vitamin B12, Vitamin C, Vitamin A, Vitamin D, Vitamin E, Pilocarpin, Aluminiumaminoacetat, Cimetidine, Esomeprazole, Famotidine, Lansoprazole, Magnesiumoxide, Nizatide and or Ratinidine.

The active agents to be used in connection with the present invention may be any substance desired to be released from the solid oral composition. The active agents, for which a controlled and/or accelerated rate of release is desired, are primarily substances with a limited water-solubility, typically below 10 g/100 ml inclusive of substances which are totally water-insoluble. Examples are medicines, dietary supplements, oral compositions, anti-smoking agents, highly potent sweeteners, pH adjusting agents, flavourings etc.

Other active ingredients are, for instance, paracetamol, benzocaine, cinnarizine, menthol, carvone, coffeine, chlorhexidine-di-acetate, cyclizine hydrochloride, 1, 8- cineol, nandrolone, miconazole, mystatine, aspartame, sodium fluoride, nicotine, saccharin, cetylpyridinium chloride, other quaternary ammoniumcompounds, vitamin E, vitamin A, vitamin D, glibenclamide or derivatives thereof, progesterone, acetyl- salicylic acid, dimenhydrinate, cyclizine, metronidazole, sodium hydrogencarbonate, the active components from ginkgo, the active components from propolis, the active components from ginseng, methadone, oil of peppermint, salicylamide, hydrocortisone or astemizole.

Examples of active agents in the form of dietary supplements are for instance salts and compounds having the nutritive effect of vitamin B2 (riboflavin), B12, folinic acid, niacine, biotine, poorly soluble glycerophosphates, amino acids, the vitamins A, D, E and K, minerals in the form of salts, complexes and compounds containing calcium, phosphorus, magnesium, iron, zinc, copper, iodine, manganese, chromium, selenium, molybdenum, potassium, sodium or cobalt.

Furthermore, reference is made to lists of nutrients acccepted by the authorities in different countries such as for instance US code of Federal Regulations, Title 21, Section 182. 5013.182 5997 and 182.8013-182. 8997.

Examples of active agents in the form of compounds for the care or treatment of the oral cavity and the teeth, are for instance bound hydrogen peroxide and compounds capable of releasing urea during chewing.

Examples of active agents in the form of anti-smoking agents include for instance: nicotine, tobacco powder or silver salts, for instance silver acetate, silver carbonate and silver nitrate.

Further examples of active agents are medicines of any type.

Examples of active agents in the form of medicines include coffeine, salicylic acid, salicyl amide and related substances (acetylsalicylic acid, choline salicylate, mag- nesium salicylate, sodium salicylate), paracetamol, salts of pentazocine (pentazocine hydrochloride and pentazocinelactate), buprenorphine hydrochloride, codeine hydro- chloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int., Nov. 85, pages 267-271, Barney H. Hunter and Robert L. Talbert, nitroglycerine, erythrityl tetranitrate, strychnine and salts thereof, lidocaine, tetracaine hydrochloride, etorphine hydrochloride, atropine, insulin, enzymes (for instance papain, trypsin, amyloglucosidase. glucoseoxidase, streptokinase, streptodornase, dextranase, alpha amylase), polypeptides (oxytocin, gonadorelin, (LH. RH), desmopressin acetate (DDAVP), isoxsuprine hydrochloride, ergotamine compounds, chloroquine (phosphate, sulfate), isosorbide, demoxytocin, heparin.

Other active ingredients include beta-lupeol, Letigene, Sildenafil citrate and derivatives thereof.

Dental products include Carbamide, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, Fluor containing ingredients, Fluorides, Sodium fluoride, Aluminium fluoride Ammonium fluoride, Calcium fluoride, Stannous fluoride, Other fluor containing ingredients Ammonium fluorosilicate, Potasium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl Propylenediamine Dihydrofluoride, Vitamins include A, Bl, B2, B6, B12, Folin acid, niacin, Pantothensyre, biotine, C, D, E, K. Minerals include Calcium, phosphor, magnesium, iron, Zink, Cupper, Iod, Mangan, Crom, Selene, Molybden. Other active ingredients include: Q10@, enzymes.

Natural drugs including Ginkgo Biloba, ginger, and fish oil.

The invention also relates to use of migraine drugs such as Serotonin antagonists: Sumatriptan, Zolmitriptan, Naratriptan, Rizatriptan, Eletriptan; nausea drugs such as Cyclizin, Cinnarizin, Dimenhydramin, Difenhydrinat ; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid. In a preferred embodiment the invention relates to the release of Nicotine and its salts.As above mentioned active ingredients and/or flavours may be premixed into the gum base.

In the presence of an active ingredient the solid oral composition may preferably also comprise a carrier known in the art.

The solid oral compositions according to the invention may comprise warming agents. Warming agents may be selected from a wide variety of compounds known to provide the sensory signal of warming to the individual user. These compounds offer the perceived sensation of warmth, particularly in the oral cavity, and often enhance the perception of flavours, sweeteners and other organoleptic components. Useful warming agents include those having at least one allyl vinyl component, which may bind to oral receptors.

Examples of suitable warming agents include, but are not limited to: vanillyl alcohol n butylether (TK-1000, supplied by Takasago Perfumery Company Ltd., Tokyo, Japan); vanillyl alcohol n-propylether; vanillyl alcohol isopropylether; vanillyl alcohol isobutylether; vanillyl alcohol n-arninoether; vanillyl alcohol isoamylether; vanillyl alcohol n- hexylether; vanillyl alcohol methylether; vanillyl alcohol ethylether; gingerol; shogaol; paradol; zingerone; capsaicin; dihydrocapsaicin, nordihydrocapsaicin; homocapsaicin; homodihydrocapsaicin; ethanol; isopropyl alcohol; iso-amylalcohol; benzyl alcohol; glycerine; chloroform; eugenol; cinnamon oil; cinnamic aldehyde; phosphate derivatives thereof; and combinations thereof.

The warming agent generally may be present in amounts of about 0.1% to about 96% by weight of the warming composition. In some embodiments, the warming agent is present in amounts of about 5% to about 25% by weight of the composition.

In some embodiments of the present invention, the warming agent may be dispersed in pectin to form the non- particulate matrix. In such embodiments, pectin may be present in amounts of about 0.05% to about 99% by weight, more desirably about 0.05% to about 10% by weight. The warming agent may be present in amounts of about 0.005% to about 96% by weight, more desirably about 0.005% to about 10% by weight.

In some particular embodiments, the warming composition may include a warming agent present in amounts of about 0.1 % to about 99.9% by weight of the composition and a hydrated pectin matrix present in amounts of about 0.1 % to about 99.9% by weight of the composition.

In some embodiments, the warming composition may include a mixture of a warming agent and carrier (alcohol, oil and/or aqueous solvents) present in amounts of about 0.1 % to about 99.9% by weight of the composition and a hydrated food-grade polymer present in amounts of about 0.1 % to about 99.9% by weight of the composition.

In some embodiments, the warming composition may include optional additives such as flavour agents (flavours, flavourings), sweetening agents (sweeteners), coloring agents (colorants, colorings), enhancement components, and the like, and mixtures thereof.

Enhancement components may be added to enhance the perception of warmth to the user. The addition of enhancement components allows the warming agent to be present at lower levels without compromising the intensity of warmth. Such components include, for example, ionic enhancement components and/or physiological cooling agents. Ionic enhancement components include mineral cations, such as, magnesium, sodium, calcium, potassium, aluminum; phosphorous and combinations thereof. The ionic enhancement component functions by changing the ionic concentration of the user's saliva and subsequently enhancing diffusion through the mucosal membranes. Because oral receptors are highly ion sensitive, the message of warmth is delivered to the brain more rapidly than in the absence of such ionic components.

The solid oral compositions according to the invention may comprise cooling agents. Physiological cooling agents also may enhance the sensation of warmth perceived by the user. A variety of well-known cooling agents may be employed. For example, among the useful cooling agents are included menthol, xylitol, erythritol, menthane, menthone, menthyl acetate, menthyl salicylate, N,2,3-trimethyl-2-isopropyl butanamide (WS-23), N ethyl-p-menthane-3-carboxamide (WS-3), menthyl succinate, 3,1-menthoxypropane 1,2-diol and glutarate esters, among others, and combinations thereof. These and other suitable cooling agents are further described in the following U.S. patents, all of which are incorporated in their entirety by reference hereto: U.S. 4,230,688 and 4,032,661 to Rowsell et al.; 4,459,425 to Amano et al.; 4,136,163 to Watson et al.; and 5,266,592 to Grub et al. Delivery Systems Some embodiments of the present invention provide delivery systems for the oral warming compositions described above. The delivery systems (also referred to as "comestibles") generally encompass any edible or consumable compositions, such as foods and beverages. More particularly, the comestible may be selected from forms such as, but not limited to, hard candy, soft candy, cotton candy, pressed tablets, chewing gum, film, lozenges, center-filled confectioneries, such as gel or liquid filled confectioneries in any shape, liquid beverages, powdered beverages, and the like. Such comestibles include an oral warming composition, a flavour agent and a carrier.

Several different processes for manufacturing of chewing gum are known within the art. The different processes may be overall categorized in basically two different processes, that is i) chewing gum ingredients (i.e. the chewing gum base and the chewing gum additives and additional ingredients as mentioned above) are mechanically mixed on the basis of the gum base compounds or ii) chewing gum ingredient are compressed on the basis of more or less discrete gum base particles. The first type of chewing gum generally benefits of a very comfortable texture, among several different parameters, most likely due to the mechanically mixing of the polymers and for example the flavours. One disadvantage of such type of process and chewing gum is however, that the different ingredients, such as encapsulated flavour, active ingredients, etc. may be more or less destroyed or degraded by the mixing process.

The second type of chewing gum generally benefits of a relatively gentle handling of vulnerable additives, such as the above mentioned flavours or active ingredients. One disadvantage of such type of chewing gum is however, that the resulting chewing gum tablet may typically disintegrate to easy, especially during the initial chew of the gum. The pre-mixing of flavours or active ingredients may e. g. be performed by means of conventional mixers, e. g. a Z-blade mixer, during no or preferably relatively little added heating and substantially under atmospheric pressure. Preferably, the pre- mixing (also referred to a tearing) should be purely mechanically should be performed sufficiently enough to result in a homogeneous blend of the flavour and/or active ingredients into the gum base.

Typical duration in time of mixing may be between few minutes op to e. g. 30 minutes. Evidently, according to the invention, other temperatures, pressures, duration in time and mixing methods may be applied for the purpose of mixing active ingredients and/or flavours into the gum base and thereby the gum base granulate applied for the subsequent compression.

Chewing gums according to the invention are not restricted to type and may be of any type such as conventional chewing gum, liquid filled chewing gum or compressed chewing gum.

Accordingly, in one embodiment according to the invention the chewing gum is formulated as a conventional chewing gum.

In one embodiment according to the invention the chewing gum is formulated as a liquid filled chewing gum, preferably a liquid filled chewing gum.

In one embodiment according to the invention the chewing gum is formulated as a toffee imitating chewing gum.

In one embodiment according to the invention the chewing gum is formulated as a compressed chewing gum tablet.

In another embodiment, the compositions of the invention are formulated as confectionary compositions comprising a confectionary base, said confectionary base preferably comprising from 0% to 99%, particularly from 15% to 98%, preferably 30% to 97% by weight of the composition. Accordingly, in this embodiment the invention is a solid oral composition comprising:

i) a confectionary base

ii) rod-shaped apatite crystals of the formula $Ca_5(PO_4)_3(OH)_xF_y$ having the following features i) the length-to-breadth ratio of the crystals is at least $\geq 5$ and ii) x+y=1, where if x or y$\neq$0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions.

Non-limiting examples of confectionary compositions according to the invention include lozenges, high boiling, grained sugar confectionary, chocolate, compressed tablets, and soft candy such as gummy confectionary and jellies.

Preferred embodiments of confectionary compositions according to the invention are contemplated, said preferred embodiments being derived from the corresponding chewing gum embodiments.

The compositions according to the invention, particularly the chewing gum compositions according to the invention may be formulated as sticks or pellets, and may be coated with a suitable coating.

In one embodiment according to the invention, the solid oral composition comprises about 1 to about 75% by weight of an outer coating applied onto the solid oral composition. In the present context, a suitable outer coating is any coating that results in extended storage stability of the solid oral composition products as defined above, relative to a solid oral composition of the same composition that is not coated. Thus, suitable coating types include hard coatings, film coatings and soft coatings of any composition including those currently used in coating of chewing gum, pharmaceutical products and confectioneries.

According to a preferred embodiment of the invention, a film coating is applied to the compressed chewing gum tablet.

One presently preferred outer coating type is a hard coating, which term is used in the conventional meaning of that term including sugar coatings and sugar-free (or sugar-less) coatings and combinations thereof. The objects of hard coating are to obtain a sweet, crunchy layer which is appreciated by the consumer and to protect the gum centres for various reasons as. In a typical process of providing the chewing gum centres with a protective sugar coating the gum centres are successively treated in suitable coating equipment with aqueous solutions of crystallisable sugar such as sucrose or dextrose, which, depending on the stage of coating reached, may contain other functional ingredients, e. g. fillers, colours, etc. In the present context, the sugar coating may contain further functional or active compounds including flavour compounds, pharmaceutically active compounds and/or polymer degrading substances.

In the production of solid oral composition it may, however, be preferred to replace the cariogenic sugar compounds in the coating by other, preferably crystallisable, sweetening compounds that do not have a cariogenic effect. In the art such coating are generally referred to as sugarless or sugar-free coatings. Presently preferred non- cariogenic hard coating substances include polyols, e. g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively.

In a typical hard coating process as it will be described in details in the following, a syrup containing crystallisable sugar and/or polyol is applied onto the gum centres and the water it contains is evaporated off by blowing with warm, dry air. This cycle must be repeated several times, typically 10 to 80 times, in order to reach the swelling required. The term "swelling" refers to the increase in weight of the products, as considered at the end of the coating operation by comparison with the beginning, and in relation to the final weight of the coated products. In accordance with the present invention, the coating layer constitutes about 1 to about 75% by weight of the finished solid oral composition element, such as about 10 to about 60% by weight, including about 15 to about 50% by weight.

In further useful embodiments the outer coating of the solid oral composition element of the invention is an element that is subjected to a film coating process and which therefore comprises one or more film-forming polymeric agents and optionally one or more auxiliary compounds, e. g. plasticizers, pigments and opacifiers. A film coating is a thin polymer-based coating applied to a solid oral composition centre of any of the above forms. The thickness of such a coating is usually between 20 and 100 um.

Generally, the film coating is obtained by passing the solid oral composition centres through a spray zone with atomised droplets of the coating materials in a suitable aqueous or organic solvent vehicle, after which the material adhering to the gum centres is dried before the next portion of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e. g. methylacrylate aminoester copolymer or mixtures of cellulose derivatives and acrylic polymers. A particular group of film-coating polymers, also referred to as functional polymers are polymers that, in addition to its film-forming characteristics, confer a modified release performance with respect to active components of the solid oral composition formulation. Such release modifying polymers include methylacrylate ester copolymers, ethylcellulose (EC) and enteric polymers designed to resist the acidic stomach environment, yet dissolve readily in the duodenum. The latter group of polymers include: cellulose acetate phtalate (CAP), polyvinyl acetate phtalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments, the film coating layer of the solid oral composition elements according to the invention comprises a plasticizing agent having the capacity to alter the physical properties of a polymer to render it more useful in performing its function as a film-forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity. In the present context, suitable plasticizers include polyols such as glycerol, propylene glycol, polyethylene glycol, e. g. the 200-6000 grades hereof, organic esters such as phtalate esters, dibutyl sebacate, citrate esters and thiacetin, oils/glycerides including castor oil, acetylated monoglycerides and fractionated coconut oil.

The choice of film-forming polymer (s) and plasticizing agent (s) for the outer coating of the present solid oral composition element is made with due consideration for achieving the best possible barrier properties of the coating in respect of dissolution and diffusion across the film of moisture and gasses.

The film coating of the solid oral composition elements may also contain one or more colorants or opacifiers. In addition to providing a desired colour hue, such agents may contribute to protecting the compressed gum base against pre-chewing reactions, in particular by forming a barrier against moisture and gasses. Suitable colourants/pacifiers include organic dyes and their lakes, inorganic colouring agents, e. g. titanium oxide and natural colours such as e. g. p-carotene.

Additionally, film coatings may contain one or several auxiliary substances such as flavours and waxes or saccharide compounds such as polydextrose, dextrins including maltodextrin, lactose, modified starch, a protein such as gelatine or zein, a vegetable gum and any combination thereof.

It is also an aspect of the present invention that the outer coating of the solid oral composition element can contain one or more pharmaceutically or cosmetically components including those mentioned hereinbefore.

Accordingly, in further embodiments, the above hard-coated or film-coated solid oral composition element of the invention is an element where the outer coating comprises at least one additive component selected from a binding agent, a moisture absorbing component, a film forming agent, a dispersing agent, an antisticking component, a bulking agent, a flavouring agent, a colouring agent, a pharmaceutically or cosmetically active component, a lipid component, a wax component, a sugar and an acid. If it is desired to defer the effect of any of these additive components in the outer coating until mastication of the solid oral composition, such components may, in accordance with the invention be encapsulated using any conventional encapsulation agent such as e. g. a protein including gelatine and soy protein, a cellulose derivative including any of those mentioned above, a starch derivative, edible synthetic polymers and lipid substances, the latter optionally in the form of liposome encapsulation.

In other embodiments, the solid oral composition element according to the invention is provided with an outer coating in the form generally described in the art as a soft coating. Such soft coatings are applied using conventional methods and may advantageously consist of a mixture of a sugar or any of the above non-cariogenic, sugar-less sweetening compounds, and a starch hydrolysate.

Again, it should be noted that the above-described coating is optional or that it may be postponed until it fits into the last part of the manufacturing process due to the fact that the applied barrier layer is also acting as a complete or at least a partial barrier to transfer of humidity from the environment into the tablet.

The compositions according to the present invention may also be encapsulated.

An advantage of encapsulating the agents comprised by the invention may be to obtain an increased stability of the agents, thus lending a longer storage life at a greater range of storage conditions to the compositions of the invention.

Any standard method giving partial or full encapsulation can be used for encapsulation. Suitable methods include, but are not limited to, spray drying, spray chilling, fluid-bed coating, and coacervation. These methods can be used individually or in any combination in a single step process or multiple step process.

Generally, compositions of high organic solubility, good film forming properties, and low water solubility, provide a suitable encapsulation. These compositions include acrylic polymers and copolymers, carboxyvinyl polymers, polyamides, polystyrene, polyvinyl acetate, polyvinyl acetate phthalate, polyvinyl pyrrolidine, and waxes.

However, only food grade materials should be used for the encapsulation. Two standard food grade coating materials, which are good formers, but not water soluble, are shellac and Zein. Others which are more water soluble, but also good film formers, are materials such as agar, alginates, a wide range of cellulose derivatives like ethyl cellulose and hydroxypropylmethyl cellulose, dextrin, gelatin and modified starches. It is also possible to use other encapsulants like acacia or maltodextrin for encapsulation.

In yet another embodiment of the invention, it may be desirable to include a supplement, such as vitamins and/or minerals in the composition according to the invention. Vitamins are preferably added in concentrations of between 10 % - 100% of the recommended daily allowance (RDA).

Especially vitamin C may be added to the compositions of the invention.

It may be desirable to include urea in the compositions of the invention. Urea may be added as a plaque acid neutralising agent. Usually urea is added to chewable compositions in between 0.15% and 25%, particularly between 0.4% and 10%, preferably between 0.8% and 5.0%, even more preferably between 1.5 % and 2.5% by weight.

The solid oral composition according to the invention may further comprise a barrier layer. The barrier layer is basically multifunctional according to a preferred embodiment of the invention. One function is that the applied barrier layer is to protect against undesired sticking. In other words, the layer functions as a kind of lubricant layer. A further, very advantageous feature of the barrier layer is that the outer contacting surface, i. e. the surface of the solid oral composition, the consumer/user of the chewing typically needs to touch is made less sticky, thereby preventing undesired sticking to the fingers.

Generally, the above mentioned advantages may be summed up to be that stickiness may further be obtained in chewing gums made by compression and not only by the traditional mixing methods.

Moreover, according to the invention, the applied barrier layer may form or form part of a humidity barrier. Due to the fact that relatively low water content is preferred according to an embodiment of the invention, the tablet should preferably be protected against too much absorption of humidity from the air.

The applied barrier layer may comprise e. g. lubricants, anti-adherents and glidants. Magnesium stearate may e. g. be applied as a pulverized parting compound.

When the barrier layer comprises metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof, a further advantageous embodiment of the invention has been obtained.

The barrier layer may be added to the final tablet for example by depositing dosed quantities of pulverized lubricants and parting compounds on the materials contacting surfaces of pressing tools of tabletting machines.

In another embodiment, the present invention relates to the use of the compositions according to the invention to whiten tooth surfaces and/or prevent discolouration of tooth surfaces. Especially, the compositions of the invention may be used to remove or prevent discolouration of teeth due to the use of tobacco-related products and/or coffee-related products.

One preferred embodiment of the invention is a method of manufacturing of a solid oral composition according to the invention.

The different processes for production of chewing gum are well-known in the art and may be overall categorized in basically two different processes, that is i) mechanically mixing chewing gum ingredients (i.e. the chewing gum base and the chewing gum additives and additional ingredients as mentioned above) ii) chewing gum ingredient are compressed on the basis of more or less discrete gum base particles.

The pre-mixing of ingredients may e. g. be performed by means of conventional mixers, e. g. a Z-blade mixer, during no or preferably relatively little added heating and substantially under atmospheric pressure. Preferably, the pre-mixing (also referred to a tearing) should be purely mechanically should be performed sufficiently enough to result in a homogeneous blend of the ingredients.

Typical duration in time of mixing may be between few minutes op to e. g. 30 minutes. Evidently, according to the invention, other temperatures, pressures, duration in time and mixing methods may be applied for the purpose of mixing ingredients.

Preferably, in one embodiment the hydroxyapatite crystals are added as powdered nano-sized crystals.

Even more preferably, when producing the solid oral compositions according to the invention, the hydroxyapatite crystals are added to the conventional chewing gum additives, prior to contact with the gum base. This minimises the contact between the hydroxyapatite crystals and the water insoluble ingredients.

In one embodiment the hydroxyapatite crystals according to the invention are added to the coating material, thereby almost avoiding contact between the hydroxyapatite crystals and the water insoluble ingredients.

The following are further preferred embodiments of the invention.

Preferably the solid oral compositions according to the invention are chewing gums. In preferred embodiments the chewing gum is formulated as a conventional chewing gum, liquid filled chewing gum, toffee imitating chewing gum or compressed chewing gum tablet.

In a preferred embodiment according to the invention the solid oral composition is a chewing gum comprising:

    a) at least one gum base system,

b) at least one chewing gum additive,

c) rod-shaped apatite crystals of the formula $Ca_5(PO_4)_3(OH)_xF_y$ having the following features i) the length-to-breadth ratio of the crystals is at least $\geq 5$ and ii) x+y=1, where if x or y$\neq$0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions,

characterised in that the concentration of said crystals is higher in the chewing gum additive part of the chewing gum than in gum base system part of the chewing gum.

In one embodiment of the invention, the thickness and the breadth of the rod-shaped apatite crystal are 0.01 to 0.02 $\mu$m and the length of the crystals is 0.1 to 0.2 $\mu$m.

In one embodiment of the invention, the concentration of the hydroxyapatite crystals in the chewing gum additive part of the chewing gum is at least 1.3 times higher than the concentration of the said crystals in the gum base system part of the chewing gum In one embodiment of the invention, the concentration of the apatite crystals in the chewing gum additive part of the chewing gum is at least 1.5 times higher than the concentration of the said crystals in the gum base system part of the chewing gum.

In one embodiment of the invention, the concentration of the apatite crystals in the chewing gum additive part of the chewing gum is at least 2.0, such as at least 2.5, 3.0, 3.5, 4.0, 4.5, 5.0, 5.5, 6.0, 7.0, 8.0, 9.0, 10.0 times higher than the concentration of said crystals in the gum base system part of the chewing gum.

In one embodiment of the invention, the gum base system part of the chewing gum is substantially free of hydroxyapatite crystals.

In one embodiment of the invention the gum base system part comprises one or more components selected from the group comprising natural or synthetic resins, natural or synthetic elastomers, fillers, softening compounds and antioxidants and colorants.

In one embodiment of the invention, the gum base system part comprises natural or synthetic resins.

In one embodiment of the invention, the natural or synthetic resin comprises biodegradable polymers, preferably a polyester polymer.

In one embodiment of the invention, the natural resin comprises rosin esters.

In one embodiment of the invention, the natural resin comprises glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins or pentaerythritol esters of rosins.

In one embodiment of the invention, the gum base system part comprises natural or synthetic resins in an amount of 3 % to 50 % by weight of the chewing gum, preferably about 4% to 30% by weight of the chewing gum.

In one embodiment of the invention, the gum base system part comprises elastomers in an amount of 0.5% to 30% by weight of the chewing gum, preferably about 5% to 25% by weight of the chewing gum.

In one embodiment of the invention, the gum base system part is encapsulated.

In one embodiment of the invention, the gum base system part is present as multiple discrete encapsulated units within the chewing gum.

In one embodiment of the invention, the chewing gum comprise one or more components selected from the group comprising bulk sweeteners, high intensity sweeteners, flavouring agents, softeners, emulsifiers, colouring agents, binding agents, acidulants, fillers, antioxidants, cooling agents, warming agents, and active substances such as pharmaceutically or biologically active substances.

In one embodiment of the invention, the chewing gum additive part comprises on or more components selected from the group comprising bulk sweeteners, high intensity sweeteners, flavouring agents, softeners, emulsifiers, colouring agents, binding agents, acidulants, fillers, antioxidants, cooling agents, warming agents, and active substances.

In one embodiment of the invention, the chewing gum additive part comprises sweeteners in the amount of from about 5 to about 95% by weight of the chewing gum, preferably about 20 to about 80% by weight, such as 30 to 60% by weight of the gum.

In one embodiment of the invention, the chewing gum additive part comprises flavouring agents in an amount of about 0.1 % to 15 % by weight of the chewing gum, preferably about 0.8 % to 5 % by weight of the chewing gum.

In one embodiment of the invention, the chewing gum additives comprises on or more active ingredients.

In one embodiment of the invention, the chewing gum comprise a coating layer. In one embodiment thereof the coating layer comprises hydroxyapatite crystals. In one embodiment thereof the hydroxyapatite crystals are only present in the coating layer.

In one embodiment of the invention, the chewing gum further comprise a barrier layer. In one embodiment thereof the barrier layer comprises magnesium stearate and/or lubricants and/or anti-adherents and/or glidants. In one embodiment thereof the barrier layer comprises a substance selected among the group comprising metallic stearates, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil and mixtures thereof.

In one embodiment of the invention, the chewing gum is formulated as a compressed chewing gum tablet. In one embodiment thereof the compressed chewing gum tablet comprises one or more layers. In one embodiment thereof at least one of the layers comprise gum base. In one embodiment thereof at least one of the layers is substantially free of gum base. In one embodiment thereof the layer substantially free of gum base comprises said apatite crystals.

In one embodiment of the invention, the compressed chewing gum tablet comprises two layers. In one embodiment thereof at least one of the layers is substantially free of gum base. In one embodiment thereof the layer substantially free of gum base comprises said apatite crystals.

In one embodiment of the invention, the compressed chewing gum tablet comprises three layers. In one embodiment thereof at least one of the layers is substantially free of gum base. In one embodiment thereof the layer substantially free of gum base comprises said apatite crystals.

In one embodiment of the invention, the chewing gum is a liquid filled chewing gum. In one embodiment thereof the liquid in said liquid filled chewing gum comprises said apatite crystals. In one embodiment thereof only the liquid in said liquid filled chewing gum comprises said apatite crystals.

A preferred embodiment of the invention is a method of providing a chewing gum comprising rod-shaped apatite crystals of the formula $Ca_5(PO_4)_3(OH)_xF_y$ having the following features i) the length-to-breadth ratio of the crystals is at least $\geq 5$ and ii) x+y=1, where if x or y$\neq$0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions, said chewing gum being capable of releasing said crystals in the oral cavity, which method comprises the steps of i) mixing said crystals with at least one chewing gum additive into a homogenous premix and ii) mixing said crystals with remaining chewing gum additives and gum base systems. In one embodiment thereof the pre-mix is produced using a dried powder form of said crystals.

One embodiment of the invention is a chewing gum comprising rod-shaped apatite crystals of the formula $Ca_5(PO_4)_3(OH)_xF_y$ having the following features i) the length-to-breadth ratio of the crystals is at least $\geq 5$ and ii) x+y=1, where if x or y$\neq$0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions, said chewing gum being capable of releasing said crystals in the oral cavity, obtainable by a method which comprises the steps of i) mixing said crystals with at least one chewing gum additives into a homogenous premix and ii) mixing said crystals with remaining chewing gum additives and gum base systems. In one embodiment thereof the pre-mix is produced using a dried powder form of said crystals.

One embodiment of the invention is use of a chewing gum according to the invention for remineralising teeth.

One embodiment of the invention is use of a chewing gum according to the invention for preventing sensitive teeth.

One embodiment of the invention is use of a chewing gum according to the invention for whitening teeth.

Examples

In the following examples the compositions are based on a standard chewing gum composition in addition to the specified ingredient. The standard chewing gum composition used herein consists essentially of the following ingredients:

| Gum base | 40 % |
|---|---|
| Sweeteners | 50 % |
| Softeners | 8 % |
| Flavour | 2 % |

The gum base composition used herein consists essentially of the following ingredients:

| Elastomer | 15 % |
|---|---|
| Natural resins | 20 % |
| PVA | 20% |
| Filler | 20% |
| Emulsifier | 5% |
| Fat | 20 % |

The general experimental design consists of the use of a specially designed mechanical mastication device to treat stained teeth with the test chewing gums (Kleber, CJ; Schimmele RG, Putt, MS, Muhler JC: A mastication device designed for the evaluation of chewing gums, J Dent Res 60: 109-114, 1981).

Effectiveness of the respective compositions in releasing the hydroxyapatite crystals was assayed in terms of amount of calcium released from the respective compositions after chewing in a mastication device as described above. Results shown are based on an average of four different measurements.

Release of calcium was calculated as follows:

$$\% \text{ release} = \text{A-B/A} * 100 \%$$

A: Content of calcium in sample core before mastication (mg/g sample)
B: Content of calcium in sample (chewing residue) after mastication (mg/g sample).

The content of calcium in sample cores and sample residues were measured by Inductive coupled plasma (ICP) technique after the samples were heated at 550 °C overnight.

Example 1

Three standard chewing gum compositions were produced containing 5% hydroxyapatite, by weight of the composition, i.e. excluding coating. In these compositions the hydroxyapatite was added a) in a dry powdered form to the chewing gum base (Gum A), b) in liquid form to the chewing gum additives (Gum B), and c) in a dry powdered form to the chewing gum additives (Gum C). Gums were produced by conventional mixing of gum base ingredients and gum additive ingredients producing the final gum.

Hydroxyapatite added as dry powdered nano-crystals were provided by BASF, Ludwig-shafen, Germany.

Results are shown in Table 1 below

| Table 1 | | | |
|---|---|---|---|
| | Gum A | Gum B | Gum C |
| Measured release of calcium | 0 | 3,1 | 22,9 |

As can be seen from this experiment hydroxyapatite should preferably be added as powdered nano-sized crystals (nano-HAP), whereas addition of hydroxyapatite in liquid form is not as beneficial. Further the crystals should preferably be added to the chewing gum additives and not to the gum base.

Example 2

Three additional chewing gum compositions were produced containing 5% hydroxyapatite by weight of the composition, i.e. excluding coating. In these compositions the hydroxyapatite was added in a dry powdered form to the chewing gum additives. The final gums deviated in the composition of the chewing gum base. The chewing gum base comprised a) a low amount of natural resin (Gum D), b) a low amount of elastomer (Gum E) and c) a high amount of elastomer (Gum F) elastomer

Results are shown in Table 2 below

| Table 2 | | | |
|---|---|---|---|
| | Gum D | Gum E | Gum F |
| Release of calcium | 0 | 7,7 | 14,9 |

Embodiments of the invention:

Embodiment 1: A chewing gum comprising:

a) at least one gum base system,

b) at least one chewing gum additive,

c) rod-shaped apatite crystals of the formula $Ca_5(PO_4)_3(OH)_xF_y$ having the following features i) the length-to-breadth ratio of the crystals is at least $\geq 5$ and ii) $x+y=1$, where if x or $y\neq 0$ the total amount of the crystals is present as a mixture of individual hydroxyapatite crystals and fluoroapatite crystals and/or as mixed crystals, such that, based on the total amount of the crystals, $(1-x)$ 100% of the hydroxide ions present if y=0 are replaced by fluoride ions,

characterised in that the concentration of said crystals is higher in the chewing gum additive part of the chewing gum than in gum base system part of the chewing gum.

Embodiment 2: The chewing gum according to embodiment 1 wherein the thickness and the breadth of the rod-shaped apatite crystals are 0.01 to 0.02 $\mu$m and the length of the crystals is 0.1 to 0.2 $\mu$m.

Embodiment 3: The chewing gum according to embodiment 1 or 2 wherein the concentration of said crystals in the chewing gum additive part of the chewing gum is at least 1.3 times higher than the concentration of the said crystals in the gum base system part of the chewing gum.

Embodiment 4: The chewing gum according to embodiment 3 wherein the concentration of said crystals in the chewing gum additive part of the chewing gum is at least 1.5 times higher than the concentration of the said crystals in the gum base system part of the chewing gum.

Embodiment 5: The chewing gum according to embodiment 4 wherein the concentration of said crystals in the chewing gum additive part of the chewing gum is at least 2.0, such as at least 2.5, 3.0, 3.5, 4.0, 4.5, 5.0, 5.5, 6.0, 7.0, 8.0, 9.0, 10.0 times higher than the concentration of said crystals in the gum base system part of the chewing gum. Embodiment 6: The chewing gum according to embodiment 5 wherein the gum base system part of the chewing gum is substantially free of said crystals.

Embodiment 7: The chewing gum according to any of the preceding embodiments wherein said hydroxyapatite crystals are present in the composition according to the invention in an amount of between 0.01 and 30 % by weight of the solid oral composition.

Embodiment 8: The chewing gum according to embodiment 7 wherein said hydroxyapatite crystals are present in the composition according to the invention in an amount of between 0.05% and 20% by weight of the solid oral composition.

Embodiment 9: The chewing gum according to embodiment 8 wherein said hydroxyapatite crystals are present in the composition according to the invention in an amount of between 0.1 % and 15 by weight of the solid oral composition.

Embodiment 10: The chewing gum according to embodiment 9 wherein said hydroxyapatite crystals are present in the composition according to the invention in an amount of between 0.5% and 10% by weight of the solid oral composition.

Embodiment 11: The chewing gum according to embodiment 10 wherein said hydroxyapatite crystals are present in the composition according to the invention in an amount of between 0.5% and 5% by weight of the solid oral composition.

Embodiment 12: The chewing gum according to any of the above embodiments, wherein said gum base system part comprises one or more components selected from the group comprising plasticizers, elastomers, fillers, softening compounds and antioxidants and colorants.

Embodiment 13: The chewing gum according to embodiment 12, wherein said gum base system part comprises plasticizers in the form of natural or synthetic resins.

Embodiment 14: The chewing gum according to embodiment 13, wherein said natural or synthetic resins comprise biodegradable polymers, preferably polyester polymers.

Embodiment 15: The chewing gum according to embodiment 14, wherein said natural resins comprise rosin esters.

Embodiment 16: The chewing gum according to embodiment 15, wherein said natural resins comprise glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins or pentaerythritol esters of rosins.

Embodiment 17: The chewing gum according to any of the above embodiments 12-16, wherein said gum base system part comprises natural or synthetic resins in an amount of 3 % to 50 % by weight of the chewing gum, preferably about 4% to 30% by weight of the chewing gum.

Embodiment 18: The chewing gum according to any of the above embodiments, wherein said gum base system part comprises elastomers in an amount of 0.5% to 30% by weight of the chewing gum, preferably about 5% to 25% by weight of the chewing gum.

Embodiment 19: The chewing gum according to any of the above embodiments, wherein said gum base system part is encapsulated.

Embodiment 20: The chewing gum according to embodiment 19, wherein said gum base system part is present as multiple discrete encapsulated units within the chewing gum.

Embodiment 21: The chewing gum according to any of the above embodiments, comprising one or more components selected from the group comprising bulk sweeteners, high intensity sweeteners, flavouring agents, softeners, emulsifiers, colouring agents, binding agents, acidulants, fillers, antioxidants, cooling agents, warming agents, and active substances such as pharmaceutically or biologically active substances.

Embodiment 22: The chewing gum according to any of the above embodiments, wherein said chewing gum additive part comprises one or more components selected from the group comprising bulk sweeteners, high intensity sweeteners, flavouring agents, softeners, emulsifiers, colouring agents, binding agents, acidulants, fillers, antioxidants, cooling agents, warming agents, and active substances.

Embodiment 23: The chewing gum according to any of the above embodiments, wherein said chewing gum additive part comprises sweeteners in the amount of from about 5 to about 95% by weight of the chewing gum, preferably about 20 to about 80% by weight, such as 30 to 60% by weight of the gum.

Embodiment 24: The chewing gum according to any of the above embodiments, wherein said chewing gum additive part comprises flavouring agents in an amount of about 0.1 % to 15 % by weight of the chewing gum, preferably about 0.8 % to 5 % by weight of the chewing gum.

Embodiment 25: The chewing gum according to any of the above embodiments, wherein said chewing gum additives comprises active ingredients.

Embodiment 26: The chewing gum according to any of the above embodiments, which further comprise a coating layer.

Embodiment 27: The chewing gum according to embodiment 26, wherein said coating layer comprises said apatite crystals.

Embodiment 28: The chewing gum according to embodiment 26, wherein said crystals are only present in the coating layer.

Embodiment 29: The chewing gum according to any of the above embodiments, which further comprise a barrier layer.

Embodiment 30: The chewing gum according to embodiment 29, wherein said barrier layer further comprises one or more substances selected among lubricants, anti-adherents and glidants.

Embodiment 31: The chewing gum according to embodiment 30, wherein said barrier layer further comprises a substance selected among the group comprising alkalimetal or alkaline-earth metal stearates, hydrogenated vegetable oils, partially hydrogenated vegetable oils, polyethylene glycols, polyoxyethylene monostearates, animal fats, silicates, silicon dioxide, or talc.

Embodiment 32: The chewing gum according to embodiment 30, wherein said barrier layer comprises a substance selected among magnesium stearates, calcium stearates, fumed silica, powdered hydrogenated cottonseed oils, hydrogenated vegetable oils, hydrogenated soya oil and mixtures thereof.

Embodiment 33: The chewing gum according to any of the above embodiments, said chewing gum being formulated as a conventional chewing gum, liquid filled chewing gum, toffee imitating chewing gum or compressed chewing gum tablet.

Embodiment 34: The chewing gum according to embodiment 33, wherein the compressed chewing gum tablet comprises one or more layers.

Embodiment 35: The chewing gum according to embodiment 34 wherein at least one of said layers comprises gum base.

Embodiment 36: The chewing gum according to embodiment 33 or 34 wherein at least one of said layers is substantially free of gum base.

Embodiment 37: The chewing gum according to any of embodiments 34-36 wherein the compressed chewing gum tablet comprises two layers.

Embodiment 38: The chewing gum according to any of embodiments 34-36 wherein the compressed chewing gum tablet comprises three layers.

Embodiment 39: The chewing gum according to any of embodiments 34-38 wherein the apatite crystals are comprised in one or more layers of the compressed chewing gum tablet.

Embodiment 40: The chewing gum according to embodiment 39 wherein the layer substantially free of gum base comprises said apatite crystals.

Embodiment 41: The chewing gum according to embodiment 33 wherein the liquid in said liquid filled chewing gum comprises said apatite crystals.

Embodiment 42: A method of providing a chewing gum comprising rod-shaped apatite crystals of the formula $Ca_5(PO_4)_3(OH)_xF_y$ having the following features i) the length-to-breadth ratio of the crystals is at least $\geq 5$ and ii) x+y=1, where if x or y≠ 0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions, said chewing gum being capable of releasing said crystals in the oral cavity, which method comprises the steps of i) mixing said crystals with at least one chewing gum additive into a homogenous premix, and ii) mixing said crystals with remaining chewing gum additives and gum base system.

Embodiment 43: The method according to embodiment 5 wherein said pre-mix is produced using a dried powder form of said crystals.

Embodiment 44: A chewing gum obtainable by the method of embodiments 42 or 43.

Embodiment 45: A use of a chewing gum according to any of embodiments 1 - 41 for remineralising teeth.

Embodiment 46: The use of a chewing gum according to any of embodiments 1 - 41 for preventing sensitive teeth.

Embodiment 47: The use of a chewing gum according to any of embodiments 1 - 41 for whitening teeth.

**Claims**

1. A chewing gum comprising:

   a) at least one gum base system,
   b) at least one chewing gum additive,
   c) rod-shaped apatite crystals of the formula $Ca_5(PO_4)_3(OH)_x(F_y$ having the following features i) the length-to-breadth ratio of the crystals is at least $\geq 5$ and ii) x+y=1, where if x or y$\neq$ 0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions,

   **characterised in that** the concentration of said crystals is higher in the chewing gum additive part of the chewing gum than in gum base system part of the chewing gum.

2. The chewing gum according to claim 1 wherein the thickness and the breadth of the rod-shaped apatite crystals are 0.01 to 0.02 $\mu$m and the length of the crystals is 0.1 to 0.2 $\mu$m.

3. The chewing gum according to claim 1 or 2 wherein the concentration of said crystals in the chewing gum additive part of the chewing gum is at least 1.3 times higher than the concentration of the said crystals in the gum base system part of the chewing gum.

4. The chewing gum according to claim 3 wherein the gum base system part of the chewing gum is substantially free of said crystals.

5. The chewing gum according to any of the preceding claims wherein said hydroxyapatite crystals are present in the composition according to the invention in an amount of between 0.01 and 30 % by weight of the solid oral composition.

6. The chewing gum according to any of the above claims, wherein said gum base system part comprises one or more components selected from the group comprising plasticizers, elastomers, fillers, softening compounds and antioxidants and colorants.

7. The chewing gum according to claim 6, wherein said gum base system part comprises plasticizers in the form of natural or synthetic resins.

8. The chewing gum according to claim 7, wherein said gum base system part comprises natural or synthetic resins in an amount of 3 % to 50 % by weight of the chewing gum, preferably about 4% to 30% by weight of the chewing gum.

9. The chewing gum according to any of the above claims, wherein said gum base system part is encapsulated.

10. The chewing gum according to any of the above claims, wherein said chewing gum additive part comprises sweeteners in the amount of from about 5 to about 95% by weight of the chewing gum, preferably about 20 to about 80% by weight, such as 30 to 60% by weight of the gum.

11. The chewing gum according to any of the above claims, which further comprise a coating layer.

12. The chewing gum according to any of the above claims, said chewing gum being formulated as a conventional chewing gum, liquid filled chewing gum, toffee imitating chewing gum or compressed chewing gum tablet.

13. A method of providing a chewing gum comprising rod-shaped apatite crystals of the formula $Ca_5(PO_4)_3(OH)_xF_y$ having the following features i) the length-to-breadth ratio of the crystals is at least $\geq 5$ and ii) x+y=1, where if x or y$\neq$ 0 the total amount of the crystals is present as a mixture of individual hydroxyapatite crystals and fluoroapatite crystals and/or as mixed crystals, such that, based on the total amount of the crystals, (1-x) 100% of the hydroxide ions present if y=0 are replaced by fluoride ions, said chewing gum being capable of releasing said crystals in the oral cavity, which method comprises the steps of i) mixing said crystals with at least one chewing gum additive into a homogenous premix, and ii) mixing said crystals with remaining chewing gum additives and gum base system.

14. A chewing gum obtainable by the method of claim 13.

**15.** A use of a chewing gum according to any of claims 1 - 14 for remineralising teeth of for preventing sensitive teeth or for whitening teeth.

**Patentansprüche**

**1.** Kaugummi, der folgendes aufweist:

a) zumindest ein Gummibasissystem,
b) zumindest einen Kaugummizusatzstoff,
c) stäbchenförmige Apatitkristalle mit der Formel $Ca_5(PO_4)_3(OH)_xF_y$ und den folgenden Merkmalen: i) das Verhältnis von Länge zu Breite der Kristalle beträgt mindestens ≥ 5 und ii) x + y = 1, wobei, falls x oder y ≠ 0 ist, die Gesamtmenge der Kristalle als Gemisch einzelner Hydroxyapatitkristalle und Fluorapatitkristalle und/ oder als gemischte Kristalle vorliegt, so dass, bezogen auf die Gesamtmenge der Kristalle, (1 - x) 100 % der vorhandenen Hydroxylionen, wenn y = 0, durch Fluoridionen ersetzt sind,

**dadurch gekennzeichnet, dass** die Konzentration der Kristalle im Anteil im Form der Kaugummizusatzstoffe des Kaugummis höher als im Anteil in Form des Gummibasissystems des Kaugummis ist.

**2.** Kaugummi nach Anspruch 1,
wobei die Dicke und die Breite der stäbchenförmigen Apatitkristalle 0,01 bis 0,02 μm und die Länge der Kristalle 0,1 bis 0,2 μm betragen.

**3.** Kaugummi nach Anspruch 1 oder 2,
wobei die Konzentration der Kristalle im Anteil in Form der Kaugummizusatzstoffe des Kaugummis mindestens 1,3 mal höher als die Konzentration der Kristalle im Anteil in Form des Gummibasissystems des Kaugummis ist.

**4.** Kaugummi nach Anspruch 3,
wobei der Anteil in Form des Gummibasissystems des Kaugummis im Wesentlichen ohne diese Kristalle ist.

**5.** Kaugummi nach einem der vorstehenden Ansprüche,
wobei die Hydroxyapatitkristalle in der erfindungsgemäßen Zusammensetzung in einer Menge von 0,01 bis 30 Gew.-% der festen oralen Zusammensetzung vorliegen.

**6.** Kaugummi nach einem der vorstehenden Ansprüche,
wobei der Anteil in Form des Gummibasissystems ein oder mehrere Komponenten aufweist, die aus der Gruppe ausgewählt sind, die Weichmacher, Elastomere, Füllstoffe, weichmachende Verbindungen und Antioxidantien und Färbemittel umfasst.

**7.** Kaugummi nach Anspruch 6,
wobei der Anteil in Form des Gummibasissystems Weichmacher in Form von natürlichen oder synthetischen Harzen aufweist.

**8.** Kaugummi nach Anspruch 7,
wobei der Anteil in Form des Gummibasissystems natürliche oder synthetische Harze in einer Menge von 3 bis 50 Gew.-% des Kaugummis, vorzugsweise von etwa 4 bis etwa 30 Gew.-% des Kaugummis aufweist.

**9.** Kaugummi nach einem der vorstehenden Ansprüche,
wobei der Anteil in Form des Gummibasissystems verkapselt ist.

**10.** Kaugummi nach einem der vorstehenden Ansprüche,
wobei der Anteil in Form der Kaugummizusatzstoffe Süßungsmittel in einer Menge von etwa 5 bis etwa 95 Gew.-% des Kaugummis, vorzugsweise von etwa 20 bis etwa 80 Gew.-%, wie z.B. 30 bis 60 Gew.-% des Gummis aufweist.

**11.** Kaugummi nach einem der vorstehenden Ansprüche,
der ferner eine Überzugsschicht aufweist.

**12.** Kaugummi nach einem der vorstehenden Ansprüche,

wobei der Kaugummi als herkömmlicher Kaugummi, mit Flüssigkeit gefüllter Kaugummi, Karamellbonbons imitierender Kaugummi oder gepresste Kaugummitablette formuliert ist.

13. Verfahren zum Herstellen eines Kaugummis, der stäbchenförmige Apatitkristalle mit der Formel $Ca_5(PO_4)_3(OH)_xF_y$ aufweist, die die folgenden Merkmale haben: i) das Verhältnis von Länge zu Breite der Kristalle beträgt mindestens $\geq 5$ und ii) x + y = 1, wobei, falls x oder y $\neq 0$ ist, die Gesamtmenge der Kristalle als Gemisch einzelner Hydroxyapatitkristalle und Fluorapatitkristalle und/oder als gemischte Kristalle vorliegt, so dass, bezogen auf die Gesamtmenge der Kristalle, (1 - x) 100 % der vorhandenen Hydroxylionen, wenn y = 0, durch Fluoridionen ersetzt sind, wobei der Kaugummi die Kristalle im Mundraum freisetzen kann,

wobei das Verfahren die folgenden Schritte aufweist: i) Mischen der Kristalle mit zumindest einem Kaugummizusatzstoff zu einem homogenen Vorgemisch und ii) Mischen der Kristalle mit den restlichen Kaugummizusatzstoffen und dem Gummibasissystem.

14. Kaugummi, der nach dem Verfahren gemäß Anspruch 13 erhalten werden kann.

15. Verwendung eines Kaugummis nach einem der Ansprüche 1 bis 14 zum Remineralisieren von Zähnen oder zur Verhinderung der Empfindlichkeit von Zähnen oder zum Bleichen von Zähnen.

**Revendications**

1. Gomme à mâcher comprenant :

a) au moins un système de base de gomme,
b) au moins un additif de gomme à mâcher,
c) des cristaux d'apatite en forme de barre de la formule $Ca_5(PO_4)_3(OH)_xF_y$ présentant les aspects suivants i) le rapport longueur-à-largeur des cristaux est au moins $\geq 5$ et ii) x + y = 1, où si x ou y $\neq 0$ la quantité totale des cristaux est présente comme un mélange de cristaux individuels d'hydroxyapatite et de cristaux de fluoroapatite et/ou comme cristaux mixtes, de telle sorte que, rapporté à la quantité totale des cristaux, (1-x) 100 % des ions hydroxyde présents si y=0 sont remplacés par des ions fluorures,

**caractérisée en ce que** la concentration desdits cristaux est supérieure dans la partie d'additif de gomme à mâcher de la gomme à mâcher à celle dans la partie du système de base de gomme de la gomme à mâcher.

2. Gomme à mâcher selon la revendication 1, dans laquelle l'épaisseur et la largeur des cristaux d'apatite en forme de barre sont de 0,01 à 0,02 $\mu$m et la longueur des cristaux est de 0,1 à 0,2 $\mu$m.

3. Gomme à mâcher selon la revendication 1 ou 2, dans laquelle la concentration desdits cristaux dans la partie d'additif de gomme à mâcher de la gomme à mâcher est au moins 1,3 fois supérieure à la concentration desdits cristaux dans la partie du système de base de gomme de la gomme à mâcher.

4. Gomme à mâcher selon la revendication 3, dans laquelle la partie du système de base de gomme de la gomme à mâcher est pratiquement exempte desdits cristaux.

5. Gomme à mâcher selon l'une quelconque des revendications précédentes, dans laquelle lesdits cristaux d'hydroxyapatite sont présents dans la composition selon l'invention dans une quantité de 0,01 à 30 % en masse de la composition orale solide.

6. Gomme à mâcher selon l'une quelconque des revendications ci-dessus, dans laquelle ladite partie du système de base de gomme comprend un ou plusieurs constituants choisis dans le groupe comprenant des plastifiants, des élastomères, des charges, des composés ramollissant et des antioxydants et des colorants.

7. Gomme à mâcher selon la revendication 6, dans laquelle ladite partie de système de base de gomme comprend des plastifiants dans la forme de résines naturelles ou synthétiques.

8. Gomme à mâcher selon la revendication 7, dans laquelle ladite partie de système de base de gomme comprend des résines naturelles ou synthétiques dans une quantité de 3 % à 50 % en masse de la gomme à mâcher, de préférence d'environ 4 % à 30 % en masse de la gomme à mâcher.

9. Gomme à mâcher selon l'une quelconque des revendications ci-dessus, dans laquelle ladite partie de système de base de gomme est encapsulée.

10. Gomme à mâcher selon l'une quelconque des revendications ci-dessus, dans laquelle ladite partie d'additif de gomme à mâcher comprend des édulcorants dans la quantité d'environ 5 à environ 95 % en masse de la gomme à mâcher, de préférence d'environ 20 à environ 80 % en masse, tel que de 30 à 60 % en masse de la gomme.

11. Gomme à mâcher selon l'une quelconque des revendications ci-dessus, laquelle comprend de plus une couche de revêtement.

12. Gomme à mâcher selon l'une quelconque des revendications ci-dessus, ladite gomme à mâcher étant formulée comme une gomme à mâcher classique, une gomme à mâcher remplie de liquide, une gomme à mâcher imitant un caramel ou une dragée de gomme à mâcher comprimé.

13. Procédé pour la fourniture d'une gomme à mâcher comprenant des cristaux d'apatite en forme de barre de la formule $Ca_5(PO_4)_3(OH)_xF_y$ présentant les aspects suivants i) le rapport longueur-à-largeur des cristaux est au moins $\geq 5$ et ii) $x + y = 1$, où si x ou $y \neq 0$ la quantité totale des cristaux est présente comme un mélange de cristaux individuels d'hydroxyapatite et de cristaux de fluoroapatite et/ou comme cristaux mixtes, de telle sorte que, rapporté à la quantité totale des cristaux, $(1-x)$ 100 % des ions hydroxyde présents si y=0 sont remplacés par des ions fluorures, ladite gomme à mâcher étant capable de libérer lesdits cristaux dans la cavité orale, lequel procédé comprend les étapes consistant i) à mélanger lesdits cristaux avec au moins un additif de gomme à mâcher en un pré-mélange homogène, et ii) à mélanger lesdits cristaux avec les additifs de gomme à mâcher restants et le système de base de gomme.

14. Gomme à mâcher pouvant être obtenue par le procédé selon la revendication 13.

15. Utilisation d'une gomme à mâcher selon l'une quelconque des revendications 1-14 pour la reminéralisation des dents ou pour la prévention des dents sensibles ou pour le blanchiment des dents.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9818719 A
- US 2004171471 A
- WO 0025598 A
- US 4230688 A
- US 4032661 A, Rowsell
- US 4459425 A, Amano
- US 4136163 A, Watson
- US 5266592 A, Grub

**Non-patent literature cited in the description**

- **Pickel FD ; Bilotti A.** The effects of a chewing gum containing dicalcium phosphate on salivary calcium and phosphate Ala. *J. Med. Sci.,* 1965, vol. 2 (3), 288-293
- **H. P. Fiedler.** Lexikon der Hilfstoffe fr Pharmacie, Kosmetik und Angrenzende Gebiete. 1981, 63-64
- **Martindale.** The Extra Pharmacopoeia. 547-578
- *J. Dent. Res.,* 1949, vol. 28 (2), 160-171
- **Barney H. Hunter ; Robert L. Talbert.** *Pharm. Int.,* November 1985, 267-271
- **Kleber, CJ ; Schimmele RG ; Putt, MS ; Muhler JC.** A mastication device designed for the evaluation of chewing gums. *J Dent Res,* 1981, vol. 60, 109-114